# EUROPÄISCHE PATENTSCHRIFT

(11) **EP 2 435 111 B1**
(45) Veröffentlichungstag und Bekanntmachung des Hinweises auf die Patenterteilung: **11.03.2015**
(21) Anmeldenummer: 10721994.1
(22) Anmeldetag: 14.05.2010
(51) Int. Cl.: A61M 5/178, A61M 5/31, A61M 5/315, A61M 5/30

(54) **ZWEIKAMMER ZYLINDER-KOLBEN-EINHEIT ZUR LYOPHILISATION, LAGERUNG, REKONSTITUTION UND APPLIKATION VON INJEKTIONSLÖSUNGEN FÜR EINEN INJEKTOR UND VERFAHREN ZUM BEFÜLLEN DER ZYLINDER-KOLBEN-EINHEIT**
DUAL-CHAMBER CYLINDER-PISTON UNIT FOR THE LYOPHILISATION, STORAGE, RECONSTITUTION AND APPLICATION OF INJECTION SOLUTIONS FOR AN INJECTOR, AND METHOD FOR FILLING THE CYLINDER-PISTON UNIT
ENSEMBLE CYLINDRE/PISTON À DEUX CHAMBRES POUR INJECTEUR, PERMETTANT LA LYOPHILISATION, LA CONSERVATION, LA RECONSTITUTION ET L'ADMINISTRATION DE SOLUTIONS INJECTABLES, ET PROCÉDÉ DE REMPLISSAGE DE L'ENSEMBLE CYLINDRE/PISTON

(30) Priorität: 29.05.2009 DE 102009023335; 18.11.2009 DE 102009053729
(43) Veröffentlichungstag der Anmeldung: 04.04.2012
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2010/002974
(87) Internationale Veröffentlichungsnummer: WO 2010/136132

(56) Entgegenhaltungen:
- EP-A2- 0 688 570
- WO-A1-2007/034020
- FR-A5- 2 082 128
- US-A1- 2004 097 874

## Beschreibung

Die Erfindung betrifft eine Zylinder-Kolben-Einheit für einen nadelfreien Injektor, beispielsweise zur Bereitstellung einer sterilen Injektionslösung, mit einer in einem Zylinder angeordneten Kammer zur Aufnahme einer Injektionslösung, einer Stirnwand mit mindestens einer Düsenbohrung bzw. einem Austrittselement und einem in der Kammer beweglich angeordneten zweiten Kolben,
- wobei die Kammer zweiteilig mit einer ersten Kammer und einer konzentrischen zweiten Kammer ausgebildet ist,
- wobei der Querschnitt der ersten Kammer größer ist als der Querschnitt der zweiten Kammer,
- wobei die erste Kammer durch einen ersten Kolben und die zweite Kammer durch den zweiten Kolben verschlossen ausgebildet ist,
- wobei die erste Kammer von der zweiten Kammer getrennt ist,

Die Erfindung betrifft darüber hinaus ein Verfahren zum entsprechenden Befüllen der Zylinder-Kolben-Einheit.

Eine Vorrichtung mit diesen Merkmalen ist beispielsweise aus der WO 2007/034020 A1 bekannt.

Zylinder-Kolben-Einheiten werden in Injektoren eingesetzt, die beispielsweise aus US 2008/014997 A1, DE 10 2007 004 211 A1 oder DE 10 2007 008 369 A1 bekannt sind.

Unter Injektionslösung werden flüssige Arzneimittel verstanden. Im Nachfolgenden sollen die verwendeten Fachbegriffe näher erläutert werden. Dem Fachmann ist der Begriff Arzneimittel bekannt. Hierunter versteht man Stoffe oder Stoffgemische für die Human- oder Tiermedizin. Sie bestehen aus dem oder den pharmazeutischen Wirkstoffen sowie weiteren üblichen Bestandteilen, die diesen Wirkstoff pharmazeutisch verwendbar machen, insbesondere Wasser.

Aus der DE 10 2005 054 600 A1 ist eine Zylinderkolbeneinheit mit einem Zylinder und einem darin geführten Kolben bekannt, wobei der Zylinder und der Kolben eine zumindest zeitweise wirkstoffbefüllbare Kammer umschließen und der Zylinder an seinem vorderen Ende mindestens ein Austrittselement aufweist. Der Querschnitt der Kammer oder der Querschnitt der Zylinderinnenwandung vergrößert sich zumindest bereichsweise von vorne nach hinten. Der Kolben weist zumindest in dem - dem Austrittselement zugewandten - vorderen Bereich eine vordere, elastische Schürze auf, deren vordere Außenkante beim unbelasteten Kolben eine Querschnittsfläche aufspannt, die größer ist als eine Fläche, die von einer Umrisslinie aufgespannt wird, die im Bereich des Übergangs von der Schürze zum schürzentragenden Kolbenabschnitt liegt.

Aus der DE 10 1006 040 888 B3 ist ein Verschlusssystem für Behältnisse zur Aufbewahrung oder Verabreichung von flüssigen, pastösen oder pulverförmigen Stoffen bekannt, das aus einer, mit einem Durchgangsloch ausgestatteten Kappe und einem Verschlusselement besteht. Dabei hält die Kappe das Verschlusselement auf dem Behältnis im Bereich der zu verschließenden Öffnung mittels eines am Behältnis vorhandenen Rastelements kraft- und/oder formschlüssig. Die die Behältnisöffnung umgebende Stirnfläche, auf der das Verschlusselement aufliegt, weist eine Vertiefung auf. Das Verschlusselement ist eine viren-, bakterien- und sporendichte Folie, die auf der Stirnfläche und zumindest bereichsweise über der Vertiefung liegt. Beim Aufsetzen der Kappe ist zwischen dem Verschlusselement und der Vertiefung ein Elastomer- oder Klebstoffring angeordnet, der die Vertiefung ausfüllt.

Aus der DE 10 2006 045 959 B3 ist eine Zylinderkolbeneinheit mit einem Zylinder und einem darin geführten, steril gummiabgedichteten Kolben bekannt, wobei der Zylinder und der Kolben eine zumindest zeitweise wirkstoffbefüllbare Kammer umschließen und der Zylinder an seinem vorderen Ende mindestens ein Austrittselement aufweist. Der in einer hinteren Position ruhende Kolben ist gegenüber dem Zylinder mit einem statischen hinteren Dichtelement steril abgedichtet, wobei beide Dichtelemente in einer Dichtstellung jeweils an der Wandung des Zylinders und jeweils an der Wandung des Kolbens anliegen. Räumlich ist hinter jedem statischen Dichtelement ein, das jeweilige Dichtelement aufnehmender, Parkraum angeordnet. Bei betätigtem Kolben werden die einzelnen statischen Dichtelemente aus ihrer jeweiligen Dichtstellung heraus in eine im Parkraum gelegene Parkstellung überführt, wobei jedes Dichtelement in der Parkstellung entweder nur die Zylinderwandung oder nur die Kolbenwandung kontaktiert. Zwischen den beiden statischen Dichtelementen ist mindestens ein dynamisches kolbenseitiges Dichtelement angeordnet, das zumindest bei betätigtem Kolben an der Zylinderinnenwandung anliegt.

Bei den bekannten Ausführungen wird die Zylinder-Kolben-Einheit entweder unmittelbar vor der Anwendung vom Benutzer des Einweginjektors mit der Injektionslösung befüllt oder die Injektionslösung wird im Herstellerwerk in die Zylinder-Kolben-Einheit gefüllt und danach auch in der Zylinder-Kolben-Einheit gelagert. Somit liegt in der Zylinder-Kolben-Einheit über den möglichen Lagerzeitraum eine fertige Injektionslösung vor. Eine mögliche bevorzugte Lagerart eines Wirkstoffes besteht darin, die Wirkstofflösung gefrierzutrocknen (lyophilisieren) und den Wirkstoff in Form eines Pulvers zu lagern. Ein derartiges Lyophilisat weist eine längere Haltbarkeit als in gelöster Form auf. Um vor der Injektion eine fertige Injektionslösung zu erhalten, wird der lyophilisierte Wirkstoff in einem Lösungsmittel wie Wasser aufgelöst. Anschließend liegt eine injizierfähige Lösung vor.

Ausgehend von diesem Stand der Technik liegt der Erfindung die Aufgabe zugrunde, eine Zylinder-Kolben-Einheit zur Aufnahme einer Injektionslösung sowie ein Verfahren zum Befüllen der Zylinder-Kolben-Einheit anzugeben, bei der die Injektionslösung aus einem ersten Bestandteil / einer ersten Komponente und einem zweiten Bestandteil / einer zweiten Komponente vorgelegt und erst kurz vor der Anwendung fertig gemischt oder gelöst wird. Weiterhin ist eine Zylinder-Kolben-Einheit zu konstruieren, in die eine gefrierzutrocknende Injektionslösung steril eingefüllt werden kann, diese dann gefriergetrocknet werden kann und danach luftdicht steril verschlossen werden kann. In Nachbarschaft dazu muss das Lösungsmittel für das Lyophilisat so aufbewahrt werden, dass es im Anwendungsfall dieses zu einer gebrauchsfertigen Injektionslösung lösen kann. Diese Lösung muss sich vor Applikation Gasblasenfrei in einem kurzzeitig bis 300 bar stabilen Zylinder mit einer steril freizugebenden Düse befinden aus der es unter hohem Druck nadelfrei durch die Haut hindurch bis z.B. ins Unterhautfettgewebe geschossen werden kann.

Diese Aufgaben werden zunächst durch die Vorrichtung des Patentanspruchs 1 gelöst. Demnach ist die einleitend bezeichnete Vorrichtung dadurch gekennzeichnet, dass der zweite Kolben mit mindestens einem Überleitungskanal oder dass die erste Kammer mit mindestens einem verschließbaren, axial ausgerichteten Gefriertrocknungskanal und die zweite Kammer mit mindestens einem axial ausgerichteten Überleitungskanal, an den jeweils hinteren Enden der Kammern und dass der zweite Kolben als ein innerer zweiter Kolbenkörper mit einem äußeren zweiten Kolbenring und der erste Kolben als ein innerer erster Kolbenkörper mit einem äußeren ersten Kolbenring ausgebildet ist.

Durch die Anordnung des zweiten Kolbens zwischen der zweiten Kammer und der ersten Kammer wird eine Trennung des ersten Bestandteils / der ersten Komponente von dem zweiten Bestandteil / der zweiten Komponente ermöglicht.

Durch eine bevorzugte Ausgestaltung der Kolben in jeweils einen Kolbenkörper und einen äußeren Kolbenring können unterschiedliche Materialien für die Kolben berücksichtig werden. Sowohl der äußere Kolbenring des ersten als auch der des zweiten Kolbens werden beispielsweise beim Einsetzen in die erste Kammer so gedrückt, dass ein Kanal bzw. eine Strömungskante entsteht, durch den die verdrängte Luft aus der ersten Kammer drucklos entweichen kann.

Zum Injizieren werden die inneren Kolbenkörper über eine Kolbenstange beispielsweise durch eine vorgespannte Feder in die zweite Kammer in kürzester Zeit eingeschoben, um die Injektionslösung unter hohem Druck aus der der/den Düse/-n gegen einen Widerstand - Haut - auszustoßen.

Eine bevorzugte Ausgestaltung sieht dabei vor, den inneren Kolbenkörper, der der/den Düse/-n am nächsten ist, mit mindestens einer Dichtlippe auszubilden, um die Führung und die Dichtigkeit zwischen innerem Kolbenkörper und der Wandung der zweiten Kammer zu verbessern und/oder druckabhängig zu erhöhen.

Die oben genannte Aufgabe wird weiterhin durch ein Verfahren, unter Berücksichtigung der soeben beschriebenen Zylinder-Kolben-Einheit, gelöst.

Das Verfahren umfasst mindestens folgende Schritte:
- Befüllen einer zweiten Kammer mit einem ersten Bestandteil / einer ersten Komponente einer Injektionslösung, wobei das Volumen des ersten Bestandteils / der ersten Komponente kleiner ist als das Volumen der zweiten Kammer;
- Einsetzen eines gequetschten, die Luft entweichen lassenden, zweiten Kolbens in eine erste Kammer bis zur Anlage an eine Ringfläche, um die zweite Kammer zu verschließen, wobei in der zweiten Kammer ein Gaspolster vorliegt;
- Befüllen der ersten Kammer oberhalb des zweiten Kolbens mit einem Lösungsmittel, z.B. Wasser oder einem zweiten Bestandteil / einer zweiten Komponente,
- Einsetzen eines ersten gequetschten Kolbens in die erste Kammer und verschließen der ersten Kammer,
- Einschieben des ersten Kolbens mit nach oben zeigender(n) Düse(n) zur Herstellung der Injektionslösung aus dem Lyophilisat bzw. dem ersten Bestandteil / der ersten Komponente und des Lösungsmittels oder des zweiten Bestandteils / der zweiten Komponente durch einen Kolbenschieber, wobei durch Überdruck in der ersten Kammer ein zweiter innerer Kolbenkörper soweit in einem zweiten äußeren Kolbenring in Richtung zweite Kammer geschoben wird, bis ein bisher verschlossener

Überleitungskanal die zweite Kammer mit der ersten Kammer verbindet und das Lösungsmittel oder der zweite Bestandteil/ die zweite Komponente vollständig in die zweite Kammer gelangt und sich mit dem Lyophilisat bzw. dem ersten Bestandteil / der ersten Komponente zur fertigen Injektionslösung löst oder mischt,
- nachfolgendes Einschieben eines ersten inneren Kolbenkörpers gemeinsam mit dem zweiten inneren Kolbenkörper mit Hilfe des Kolbenschiebers durch Eindrehen des Kolbenschiebers in ein Gewinde, Teilgewinde oder Rastelement und Herausdrücken einer Gasblase aus der Zylinder-Kolben-Einheit wobei die Gasblase durch nach oben zeigende/-n Düsenbohrung/-en bzw. Austrittselement/-e aus der zweiten Kammer verdrängt wird und eine am Außenzylinder oder an der Verschlusskappe befestigte Membrane , die sich bei Austritt des Gaspolsters von den Düsenbohrung/-en bzw. Austrittselement/-en abhebt, und
- nach dem Austritt des Gaspolsters die Düsenbohrung/-en bzw. das/die Austrittselement/-e wieder steril verschließt (Überdruckventil).

In der Regel wird die fertige Injektionslösung in die mit Verschlusskappe und Membrane verschlossene zweite Kammer steril dosiert.

In einer ersten Ausführung wird der zweite Kolben mit dem Kolbenring, der einen oben verschlossenen Kanal aufweist, der gleichzeitig als Überströmkanal und Gefriertrocknungskanal fungiert (Funktionen können an verschiedenen Stellen des Kolbenringes auch getrennt positioniert sein), so aufgesetzt, dass der gesamte Gasraum über der Injektionslösung Kontakt nach Außen hat.

In einer zweiten Ausführung wird der zweite Kolben mit dem Kolbenring so aufgesetzt, dass der gesamte Gasraum über der Injektionslösung durch mindestens einen axial ausgerichteten Gefriertrocknungskanal in der ersten Kammer Kontakt nach Außen hat. Sodann wird gefriergetrocknet. Nach dem Lyophilisieren wird der zweite Kolben mit dem Kolbenring bis zur Ringfläche vorgeschoben, so dass das Lyophilisat gegen Feuchtigkeit und Sauerstoff geschützt verschlossen ist. Danach wird das Lösungsmittel (z.B. Wasser) in die erste Kammer steril eingefüllt und mit dem ersten Kolben z.B. mit gequetschtem Kolbenring, ohne Druck auf das Lösungsmittel, verschlossen.

Eine bevorzugte Weiterbildung sieht vor, dass der Wirkstoff der Injektionslösung in der zweiten Kammer gefriergetrocknet wird.

Weitere Merkmale und Einzelheiten der Erfindung ergeben sich aus den Ansprüchen und der nachfolgenden Beschreibung eines in schematischen Zeichnungen dargestellten Ausführungsbeispiels der Erfindung. Es zeigen:
- Fig. 1.1 / 1.2: in geschnittener Seitenansicht einen Zylinder mit zwei Durchmessern und druckstabilem Außenzylinder einer Zylinder-Kolben-Einheit mit an einer Verschlusskappe montierten Membrane;
- Fig. 1.2a: in Draufsicht die Lage der Kanäle aus Figur 1.2;
- Fig. 2.1 / 2.2: in geschnittener Seitenansicht den Zylinder aus Fig. 1.1 / Fig. 1.2 mit der zu lyophilisierenden Injektionslösung und dem zweiten Kolben in einer Position, dass der Gasraum über der Injektionslösung Kontakt nach Außen hat;
- Fig. 3.1 /3.2: in geschnittener Seitenansicht den bekannten Zylinder mit dem zweiten Kolben, der das Lyophilisat steril und gasdicht verschließt;
- Fig. 4.1 / 4.2: in geschnittener Seitenansicht den Zylinder aus Fig. 3.1 / Fig. 3.2 mit einem Lösungsmittel und dem dieses gasdicht und steril verschließenden ersten Kolbens;
- Fig. 5.1 / 5.2: in geschnittener Seitenansicht den bekannten Zylinder mit geöffneten Überströmkanal, rekonstituierter Injektionslösung und Gaspolster;
- Fig. 6.1 /6.2: in geschnittener Seitenansicht den bekannten Zylinder mit durch den eingeschraubtem Kolbenschieber bis zur Luftblasenfreiheit vorgeschobenen inneren ersten und inneren zweiten Kolbenkörper;
- Fig. 7.1 / 7.2: in geschnittener Seitenansicht die injektionsbereite Zylinder-Kolben-Einheit bei entfernter Verschlusskappe und entfernter Membrane;
- Fig. 8.1 / 8.2: das System nach der Injektion mit verschlossener Düse;
- Fig. 9: in Unteransicht die Zylinder-Kolben-Einheit mit einer Düse;
- Fig. 10: in Unteransicht die Zylinder-Kolben-Einheit mit vier Düsen;
- Fig. 11: einen Querschnitt des Vier-Düsen-Modells durch zwei diagonal gegenüberliegende Düsen;
- Fig. 12: zweiter äußerer Kolbenring mit trifunktionellem Kanal;
- Fig. 13: einen Kolbenring mit trifunktionellem Kanal wie in Fig. 12, jedoch mit geringerem Totvolumen und höherer mechanischer Stabilität;
- Fig. 14: einen Kolbenring mit funktionell getrennten Gefriertrocknungskanal und Überströmkanal;
- Fig. 15: den trifunktioneller Kolbenring in alternativer Ausführung als Quadring und Schlitzring;
- Fig. 16: einen Kolbenring mit bifunktionellem Kanal;
- Fig. 17: den bifunktioneller Kolbenring aus Fig. 16 in alternativer Ausführung, als Quadring und Überleitungskanalring;
- Fig. 18: Kolbenring der Fig. 14 mit beidseitig schließendem Überströmkanal und
- Fig. 19: Kolbenring der Fig. 16 mit beidseitig schließendem Überströmkanal.

In der folgenden Beschreibung eines Ausführungsbeispiels bedeutet das vordere Ende der Zylinder-Kolben-Einheit das der Oberfläche eines Patienten am nächsten liegende Ende. Das hintere Ende der Zylinder-Kolben-Einheit ist das Ende, das von der Oberfläche des Patienten entfernt ist, wenn die Einheit angewendet wird.

In allen Figuren sind gleiche technische Elemente mit gleichen Bezugszeichen bezeichnet.

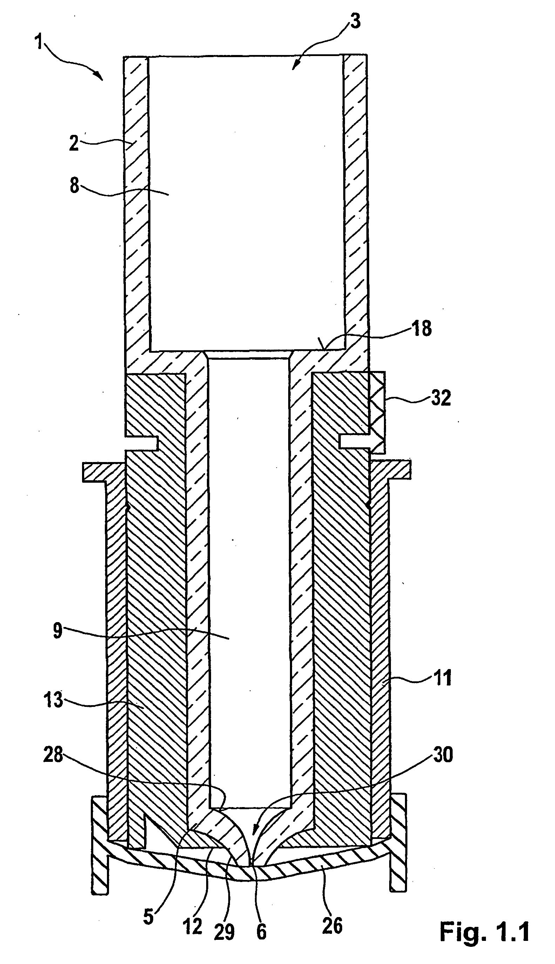

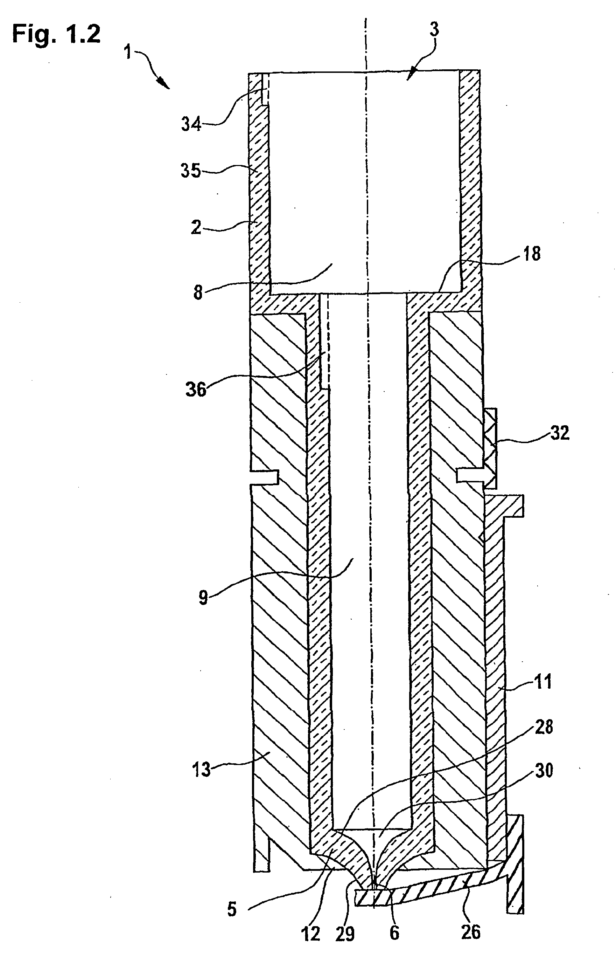

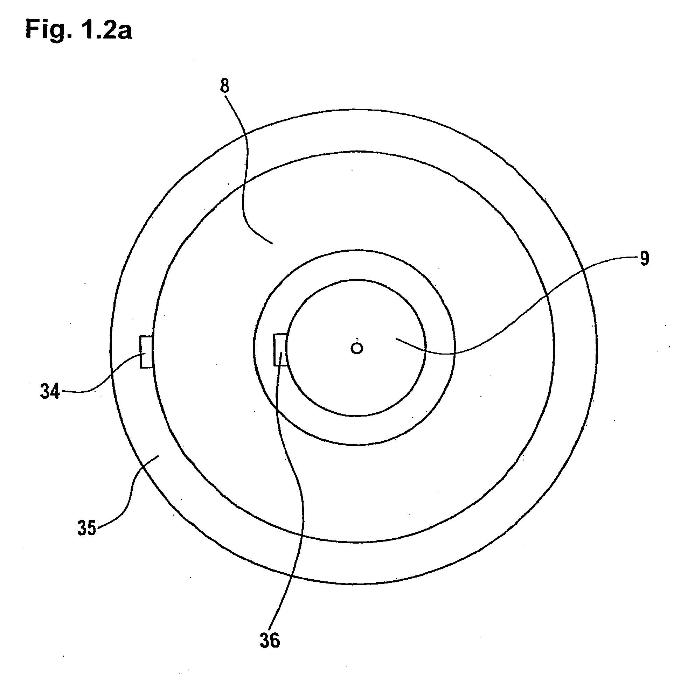

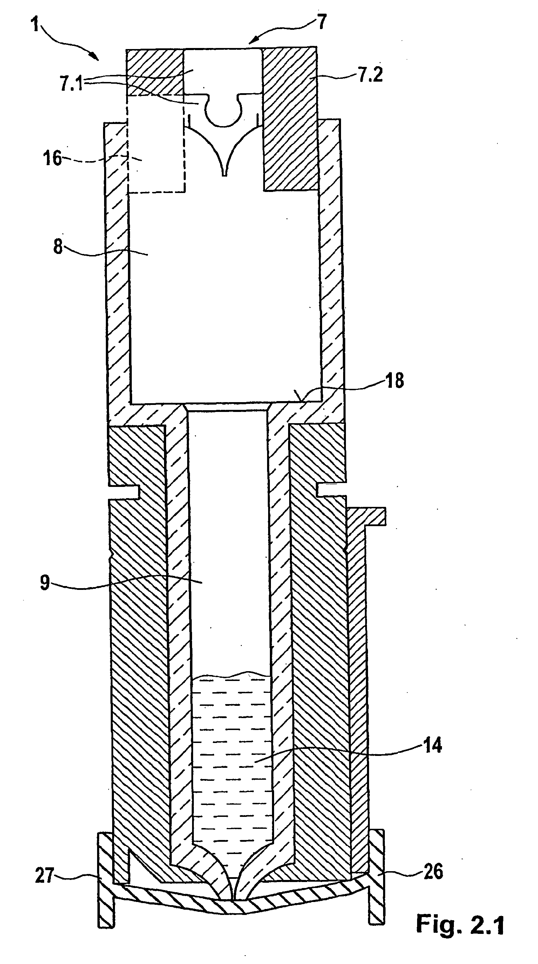

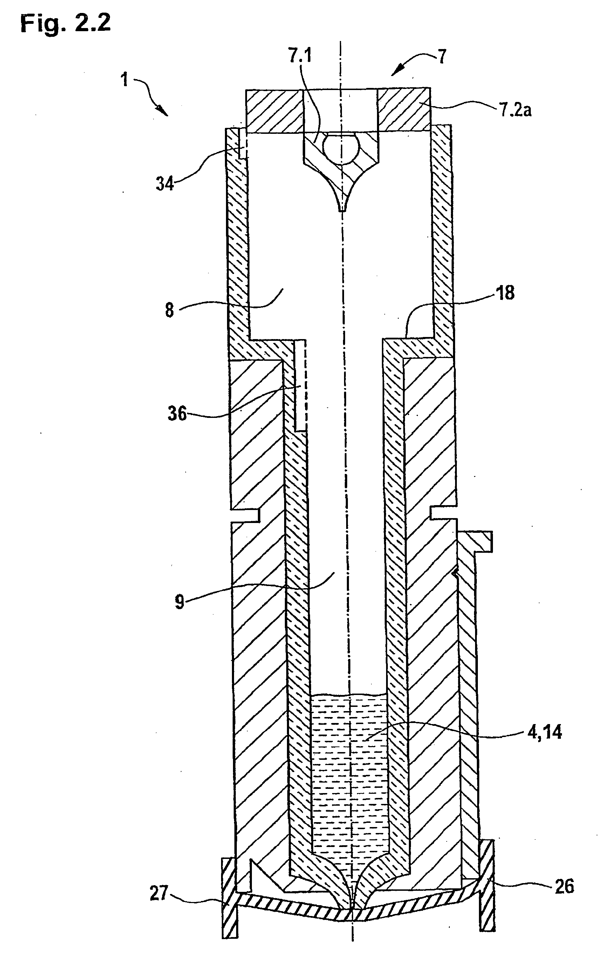

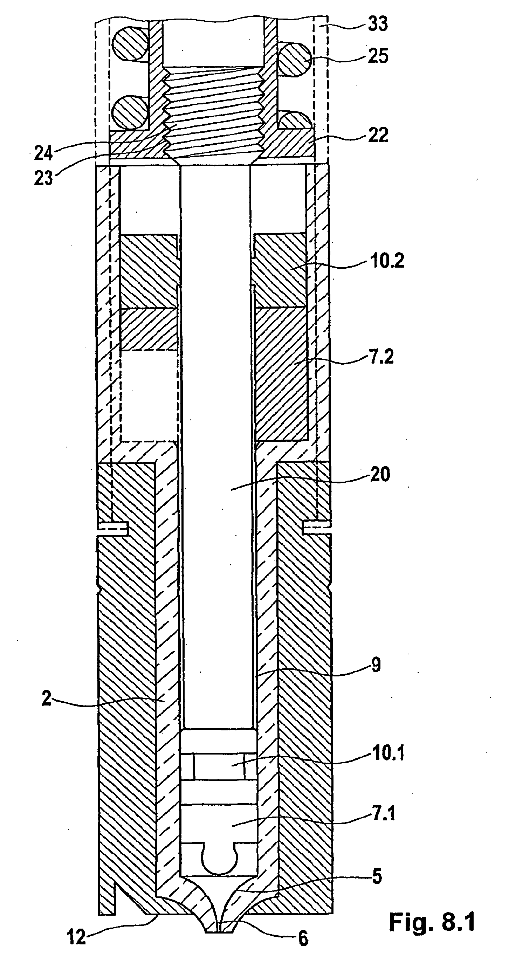

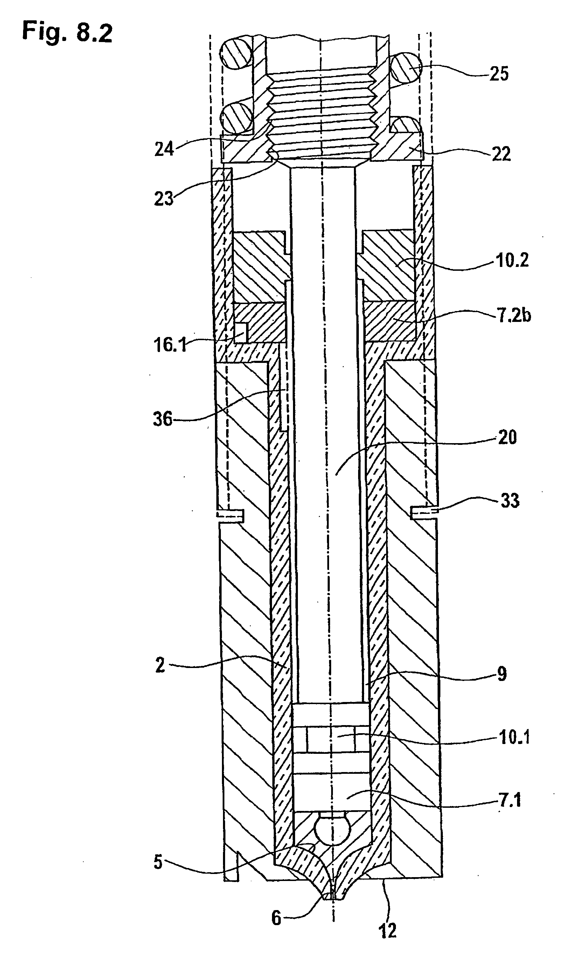

In den Figuren 1.1 und 1.2 ist in geschnittener Seitenansicht eine Zylinder-Kolben-Einheit 1 dargestellt. Eine Kammer 3 eines Wasserdampf und Sauerstoff sperrenden Zylinders 2 wird durch eine erste Kammer 8 und eine zweite konzentrische Kammer 9 gebildet, wobei der Querschnitt der ersten Kammer 8 größer ist als der Querschnitt der zweiten Kammer 9. Die zweite Kammer 9 ist an der der ersten Kammer 8 gegenüberliegende Seite mit einer Stirnwand 5 ausgebildet, die mindestens eine Düsenbohrung bzw. mindestens ein Austrittselement 6 aufweist. Jede Düsenbohrung bzw. jedes Austrittselement 6 endet an der Außenseite 12 des druckstabilen und die zweite Kammer 9 umhüllenden Außenzylinders 13 in einem erhabenen stumpfen Kegel 29 und weist an seiner Innenseite 28 der Stirnwand 5 einen Ausströmtrichter 30 auf. Auf der Außenseite 12 des druckstabilen Außenzylinders 13 befindet sich eine über das/die Austrittselemente 6 gespannte und vorzugsweise von der Verschlusskappe 11 getragene Wasserdampf und Sauerstoff sperrende Membrane 26, beispielsweise aus Gummi oder transparentem Silikon. Alternativ kann die Membrane 27 am Außenzylinder 13 montiert sein (siehe Figuren 2.1 und 2.2). Der druckstabile Außenzylinder 13 ist mit wahlweisen Halteelementen 32 wie Schlitz, Gewinde Flansch oder Bajonett ausgebildet. Beispielsweise greifen Zughaken 33 in den Schlitz (vergleiche Figuren 8.1 und 8.2).

Zwischen der ersten Kammer 8 und der zweiten Kammer 9 ist ein Übergangsbereich ausgebildet, vorzugsweise als Ringfläche 18. Eine erste Ausgestaltung des Übergangbereiches sieht dabei Radien (nicht dargestellt) beim Übergang von der zylindrischen Wandung der ersten Kammer 8 zur Ringfläche 18 und von der Ringfläche 18 zur zylindrischen Wandung der zweiten Kammer 9 vor. Eine zweite Ausgestaltung dieses Bereiches sieht Schrägen (nicht dargestellt) vor. Weitere Ausgestaltungen, auch Kombinationen von beispielsweise Radien und Schrägen, negativem oder positivem Sturz bzw. konkav oder konvex, können berücksichtigt werden. Die Ausgestaltungen helfen zum Einen beim Einfüllen der Injektionslösung 4 bzw. der ersten Komponente 14, siehe Figuren 2.1 und 2.2, und zum Anderen bei der Herstellung des Zylinders 2, der beispielsweise im Einkomponenten oder Zweikomponenten-Kunststoffspritzgießverfahren oder aus Glas hergestellt wird. Durch die oben beschriebenen Ausgestaltungen wird das Fließverhalten des berücksichtigten Wasserdampf und Sauerstoff sperrenden Materials unterstützt, um einen Zylinder 2 mit den vorgegebenen Eigenschaften wie Rechtwinkligkeit, Rundlauf, Wandstärke usw. zu erhalten.

Das für eine Injektion vorgesehene Arzneimittel wird in der Zylinder-Kolben-Einheit 1 über eine längere Zeit vorgehalten. Es ist bekannt, dass Arzneimittel bis zur Injektion in zwei Bestandteilen / Komponenten aufbewahrt werden, da z.B. die Haltbarkeit verlängert wird. Das aus einem ersten Bestandteil / einer ersten Komponente 14 und einem zweiten Bestandteil / einer zweiten Komponente 15 bestehende Arzneimittel wird kurz vor der Injektion gemischt und/oder gelöst. Bei der Komponente / dem Bestandteil 14 kann es sich um eine Flüssigkeit - wie eine Injektionslösung 4 - oder ein Pulver handeln. Eine pulverförmige Komponente / ein pulverförmiger Bestandteil ist z.B. ein Lyophilisat. Ein zweiter Bestandteil / eine zweite Komponente 15 liegt wie die Das Lösungsmittel 19 in flüssiger Form vor. Andere Formen der Komponenten / Bestandteile 14, 15 werden ausdrücklich mit berücksichtigt.

In einer alternativen Ausführung ist am hinteren Ende der Kammer 8 und der Kammer 9 ist in der Zylinderwand 35 jeweils mindestens ein Gefriertrocknungskanal 34 (in Kammer 8) und ein Überleitungskanal 36 (in Kammer 9) ausgeformt (siehe Figur 1.2)

Der Querschnitt des Gefriertrocknungskanals /der Gefriertrocknungskanäle 34 bzw. des Überleitungskanals / der Überleitungskanäle 36 liegt, vom hinteren Ende betrachtet, in Form eines Rechtecks vor. Andere Formen sind aber auch denkbar, insbesondere solche, die das Strömungsverhalten und die Festigkeit des Zylinders berücksichtigen. Siehe hierzu die Figur 1.2a.

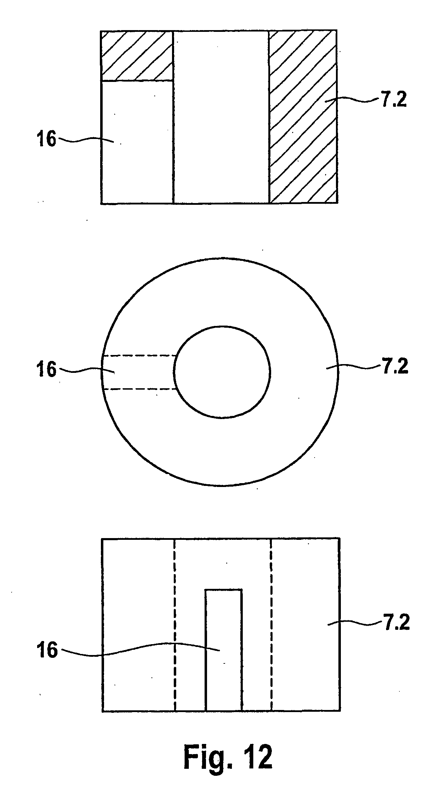

In einem ersten Arbeitsschritt, dargestellt in Figur 2.1, wird die sterile erste Komponente / der erste Bestandteil 14 in die zweite Kammer 9 des sterilen Zylinders 2 steril eingefüllt. Anschließend wird ein steriler zweiter Kolben 7 in die erste Kammer 8 derart steril eingefügt, dass ein Überleitungskanal 16 zwischen der Kammer 3 und der Umgebung offen bleibt. Zu der Ausgestaltung des Überleitungskanals 16 siehe Figuren 12 bis 19.

Alternativ wird, wie in Figur 2.2 dargestellt, ein steriler zweiter Kolben 7 in die erste Kammer 8 derart steril eingefügt, dass der Gefriertrocknungskanal 34 in der Zylinderwand 35 zwischen der Kammer 3 und der Umgebung offen bleibt.

Alle weiteren Komponenten und Arbeitsschritte sind steril, ohne dass es im weiteren Text erneut angesprochen wird.

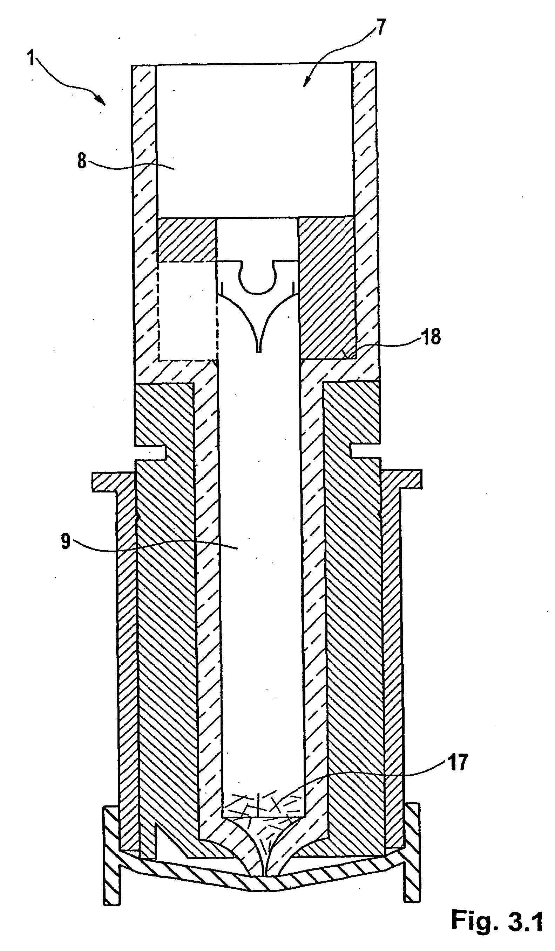

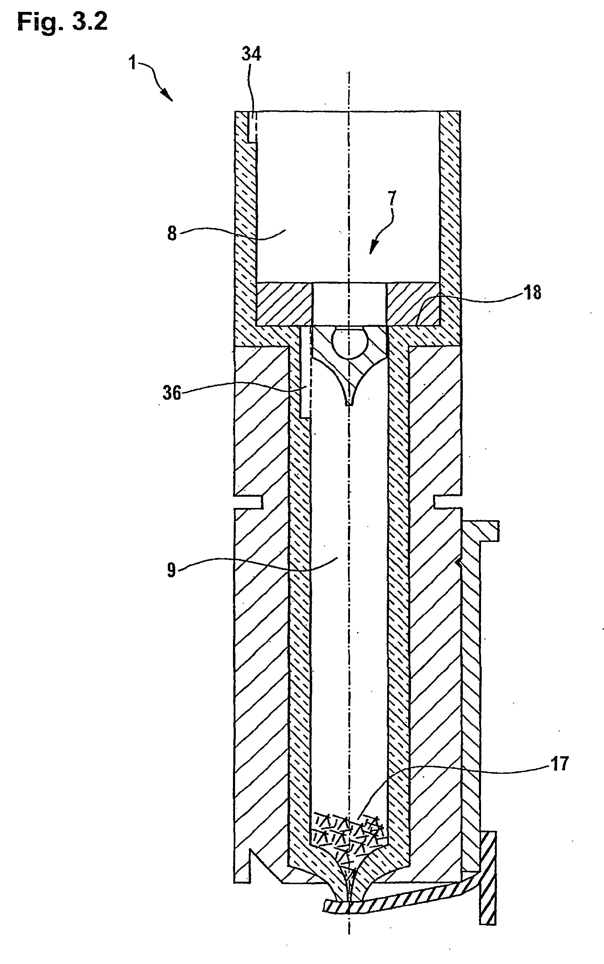

Die/der in der zweiten Kammer 9 vorgelegte Komponente / Bestandteil 14 wird nachfolgend gefriergetrocknet und verbleibt als Lyophilisat 17 in der zweiten Kammer 9, wie in den Figuren 3.1 und 3.2 dargestellt. Zum Lagern über einen längeren Zeitraum wird das Lyophilisat 17 in der zweiten Kammer 9 gas- und feuchtigkeitsdicht verschlossen. Hierzu wird der zweite Kolben 7 derart in die erste Kammer 8 eingeschoben, dass der zweite Kolben 7 an einem Übergangsbereich, der Ringfläche 18, zwischen erster Kammer 8 und zweiter Kammer 9 anliegt.

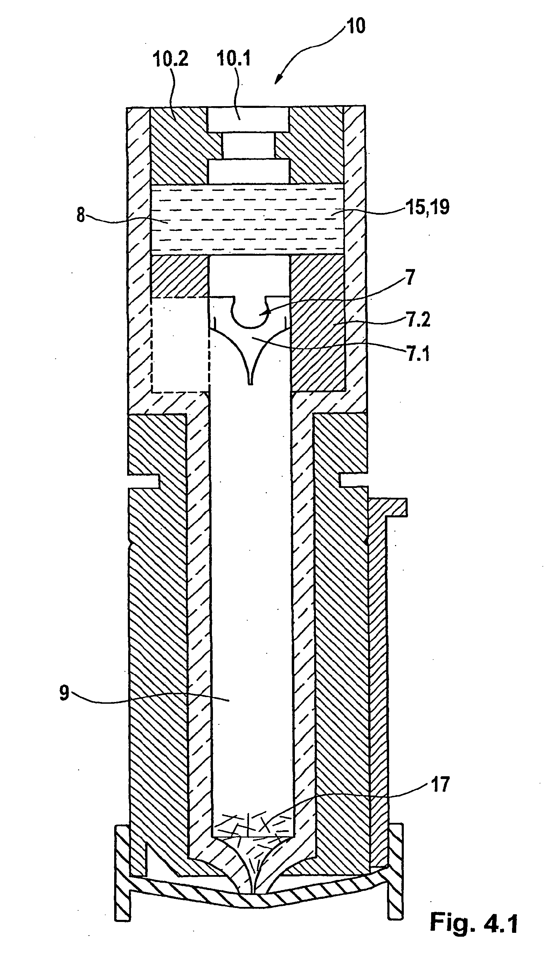

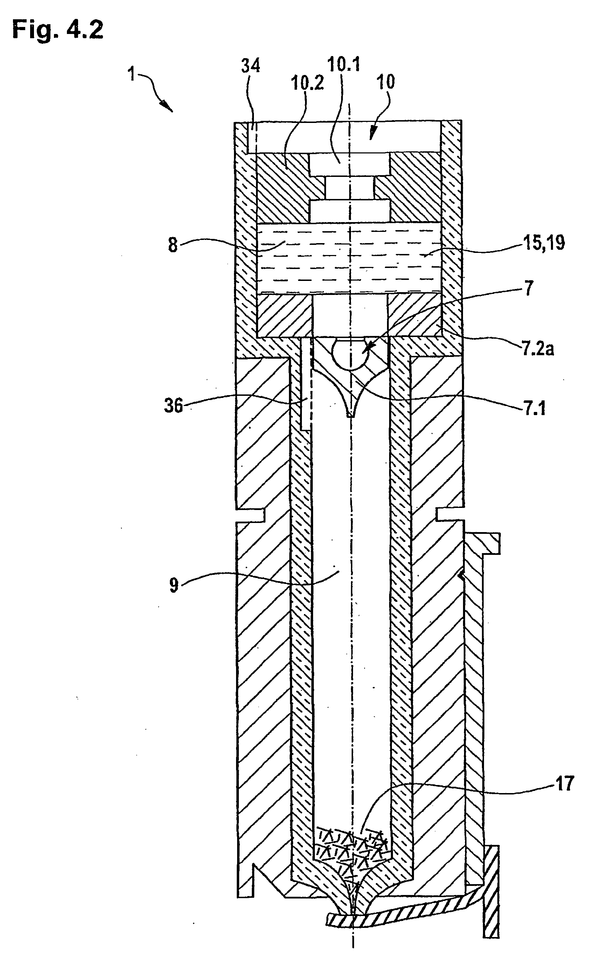

Nachdem das Lyophilisat 17, in der zweiten Kammer 9 mittels des zweiten Kolbens 7 eingeschlossen ist, wird, wie in den Figuren 4.1 und 4.2 dargestellt, oberhalb des zweiten Kolbens 7, und somit in die erste Kammer 8, der zweite Bestandteil / die zweite Komponente 15 des Arzneimittels eingefüllt. Der zweite Bestandteil / die zweite Komponente 15 ist beispielsweise ein Lösungsmittel 19. In einer bevorzugten Ausführung wird Wasser als Lösungsmittel 19 verwendet und bei Gebrauch zum Lösen des als Lyophilisat 17 vorliegenden ersten Bestandteils / ersten Komponente 14 benutzt, um ein in flüssiger Form vorliegendes Arzneimittel - die Injektionslösung 4 - injizieren zu können. Nach dem Einfüllen des zweiten Bestandteiles / der zweiten Komponente 15 in die erste Kammer 8, wird diese durch einen ersten Kolben 10 flüssigkeitsdicht verschlossen. Dabei wird der erste Kolben 10, beispielsweise durch seitliches Quetschen, drucklos in die erste Kammer 8 eingesetzt.

Sowohl der zweite als auch der erste Kolben 7, 10 werden jeweils aus mindestens einem inneren Kolbenkörper 7.1, 10.1 und einem äußeren Kolbenring 7.2, 10.2 gebildet, wobei die inneren Kolbenkörper 7.1, 10.1 in den äußeren Kolbenringen 7.2, 10.2 gleitend angeordnet sind. Die nun vorliegende Ausführungsform mit zwei getrennt verschlossenen Kammern 8, 9 dient zur Lagerung der sonst instabilen Injektionslösung 4 in Form des getrennt vorliegenden Lyophilisats 17 und dem ebenfalls getrennt vorliegenden Lösungsmittels 19.

Vor dem Injizieren der Injektionslösung 4 muss das Lyophilisat 17 mit dem Lösungsmittel 19 vermischt und das in Lösung gehen, das bis zu 30 Minuten dauern kann, abgewartet werden. Erst danach wird das verbliebene Gaspolster 31 mit nach oben zeigender(n) Düse(n) 6 herausgedrückt.

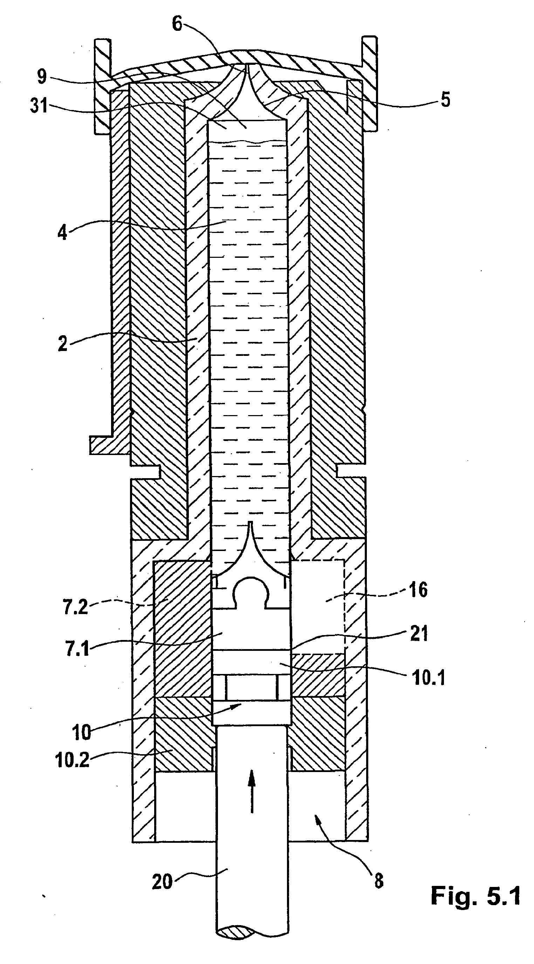

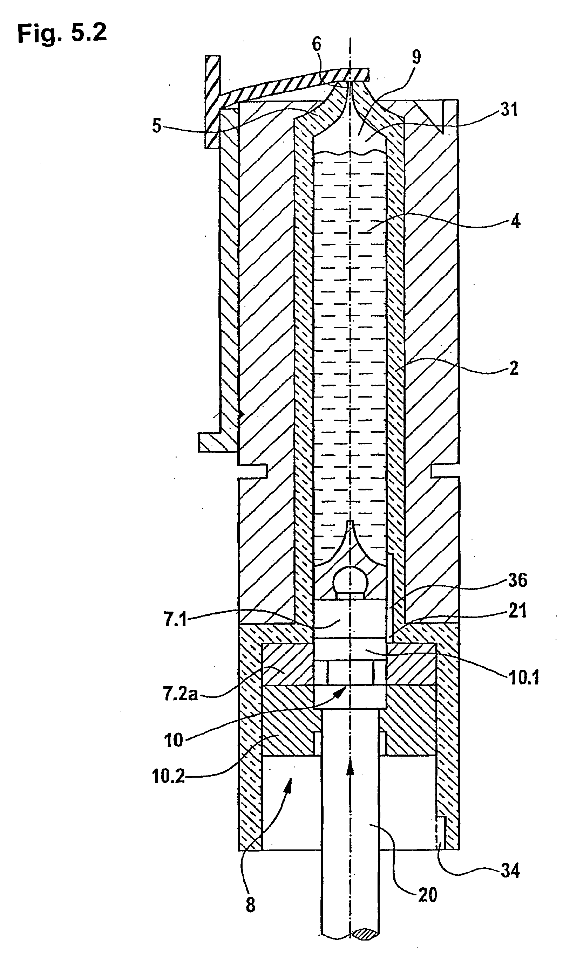

In den Figuren 5.1 und 5.2 ist dargestellt, wie das Lösungsmittel 19 aus der ersten Kammer 8 des Zylinders 2 in die zweite Kammer 9 gelangt. Hierzu wird die Zylinder-Kolben-Einheit 1, die sich in einem Injektor befindet, vorzugsweise um ca. 180° um die horizontale Achse gedreht. Dadurch steigt die Gasblase 31 in Richtung Düse bzw. Austrittselement 6. Weiterhin wird der erste Kolben 10 mittels eines Kolbenschiebers 20, der im Injektor sitzt und dort betätigt wird, in Richtung Stirnwand 5 des Zylinders 2 bewegt, wie durch den Pfeil angedeutet. Beim Einschieben verkleinert der erste Kolben 10 die erste Kammer 8, wodurch über das Lösungsmittel 19 ein Druck auf den zweiten Kolben 7 ausgeübt wird. Da der äußere Kolbenring 7.2 des zweiten Kolbens 7 auf der Ringfläche 18 aufliegt und nicht verlagert werden kann, wird durch den aufgebrachten Druck der innere Kolbenkörper 7.1 des zweiten Kolbens 7 in Richtung Stirnfläche 5 gedrückt. Bei dieser und der folgenden Manipulation muss die Düse nach oben zeigen.

In dem Augenblick, bei dem die hintere Kante 21 des inneren Kolbenkörpers 7.1 des zweiten Kolbens 7 den in dem zweiten äußeren Kolbenring 7.2 ausgebildeten Überströmungskanals 16 freigibt, fließt das Lösungsmittel 19 aus der ersten Kammer 8 in die zweite Kammer 9 und vermischt sich mit dem Lyophilisat 17 (Alternative 1).

In dem Augenblick, bei dem die hintere Kante 21 des inneren Kolbenkörpers 7.1 des zweiten Kolbens 7 den in der Zylinderwand 35 ausgebildeten Überströmungskanal 36 freigibt, fließt das Lösungsmittel 19 aus der ersten Kammer 8 in die zweite Kammer 9 und vermischt sich mit dem Lyophilisat 17 (Alternative 2).

Erst nach komplettem Lösen des Lyophilisats 17 liegt die Injektionslösung 4 zur Injektion vor. Um den Lösungsvorgang zu verbessern, ist die zweite Kammer nicht vollständig mit dem Lösungsmittel 19 und dem Lyophilisat 17 gefüllt. In der zweiten Kammer 9 befindet sich hierzu noch ein Gaspolster 31, das vor der Injektion durch weiteres Vorschieben des Kolbenschiebers 20 entfernt werden muss.

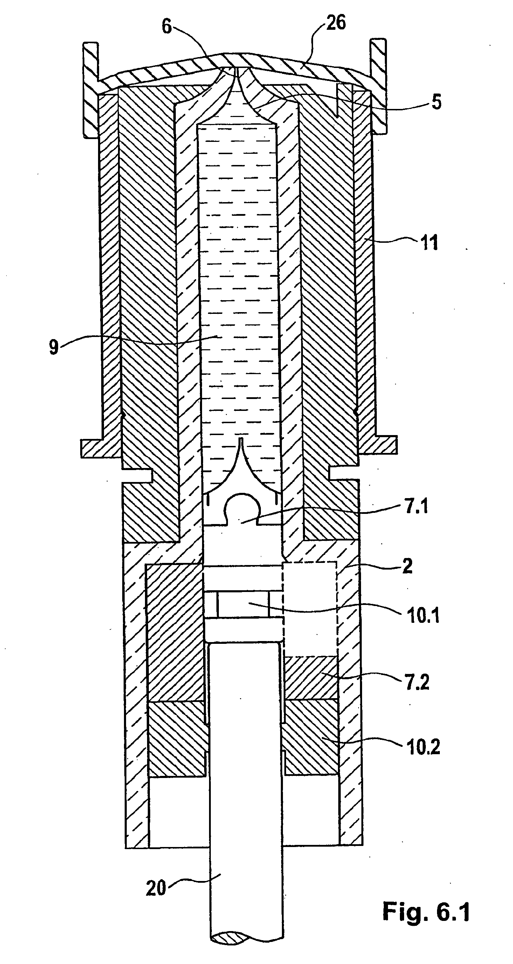

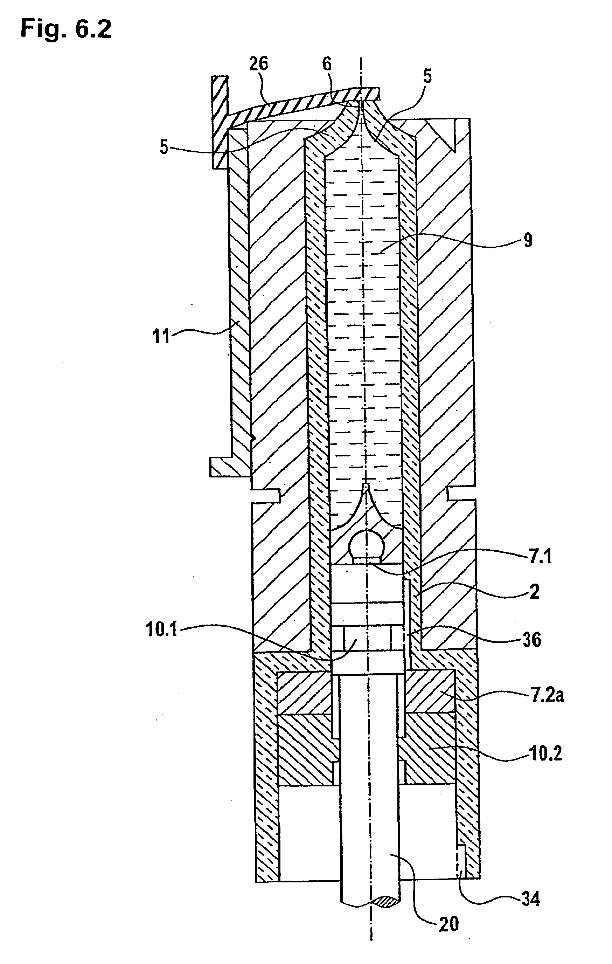

In den Figuren 6.1 und 6.2 ist dargestellt, wie der erste innere Kolbenkörper 10.1 vollständig in die erste Kammer 8 des Zylinders 2 eingeschoben ist und an dem schon komplett im Anfang der zweiten Kammer 9 steckenden zweiten inneren Kolbenkörper 7.1 anliegt. Das Lösungsmittel 19 befindet sich vollständig in der zweiten Kammer 9 als Injektionslösung 4. Der Kolbenschieber 20 des nur teilweise in Figur 8 dargestellten Injektors, ist bisher axial frei längsverschiebbar in dem Injektor angeordnet. Hierzu weist er einen Durchmesser auf, der kleiner ist als eine in einem Flansch 22 angeordnete Gewindebohrung 23 und kleiner als der Durchmesser der zweiten Kammer 9 (siehe Figuren 8.1 und 8.2). Der Kolbenschieber 20 wird axial in dem Injektor geführt und der Bereich mit dem kleineren Durchmesser ist so ausgelegt, dass bei der das Lösungsmittel 19 komplett in die Kammer 9 beförderten Position des ersten inneren Kolbenkörpers 10.1 der Bereich des Kolbenschiebers 20, der ein Gewinde 24 komplementär zur Gewindebohrung 23 aufweist, so eben in Kontakt mit dieser ist. Sodann wird durch Eindrehen der Gewinde der Kolbenschieber 20 weiter in die Zylinder-Kolben-Einheit 1 geschoben, wobei das Luftpolster entfernt wird und die Kraftübertragung des Energiespeichers auf den Kolbenschieber 20 hergestellt wird. Die Ausdrück- bzw. Ausspritz-Energie des Einweginjektors wird beispielsweise durch eine Feder 25 hervorgerufen.

Hierzu wird der erste innere Kolbenkörper 10.1 des ersten Kolbens 10 in Längsrichtung auf die Stirnwand 5 des Zylinders 2 hin geschoben, bis das Gewinde 24 des Kolbenschiebers 20 in die Gewindebohrung 23 des Flanschs 22 eingedreht werden kann. Beim Einschieben und beim nachfolgenden Eindrehen des Kolbenschiebers 20 verdrängen die aufeinander folgenden inneren ersten und zweiten Kolbenkörper 10.1 und 7.1 die Gasblase 31 aus der zweiten Kammer 9 durch die Düse bzw. das Austrittselement 6. Beim weiteren Eindrehen des Kolbenschiebers 20 entweicht die Gasblase 31 aus der zweiten Kammer 9, wobei die Verschlusskappe 11 mit einer Membrane 26 ausgebildet ist, die bei einem Überdruck in der zweiten Kammer 9 die Düse bzw. das Austrittselement 6 freigibt. Das vollständige Herausdrücken der Gasblase aus der zweiten Kammer 9 durch weiteres Einschrauben des Gewindes wird visuell vom Anwender durch gegenüberliegende Fenster (nicht dargestellt) in der Membrane 26 und durch die glasklare Verschlusskappe 11 überwacht. Auch das Einströmen des Lösungsmittels 19 kann schon mit Eindrehen des dann entsprechend längeren Gewindes des Kolbenschiebers 20 bewerkstelligt werden.

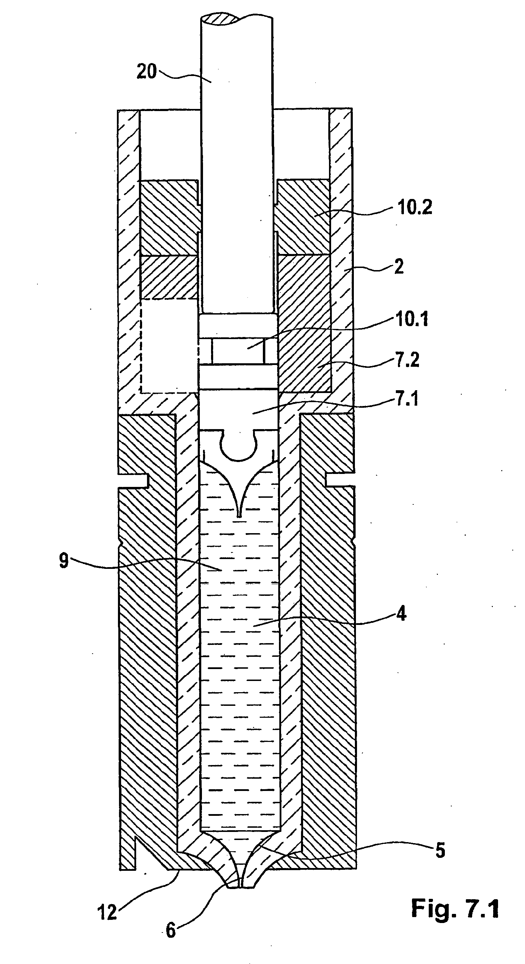

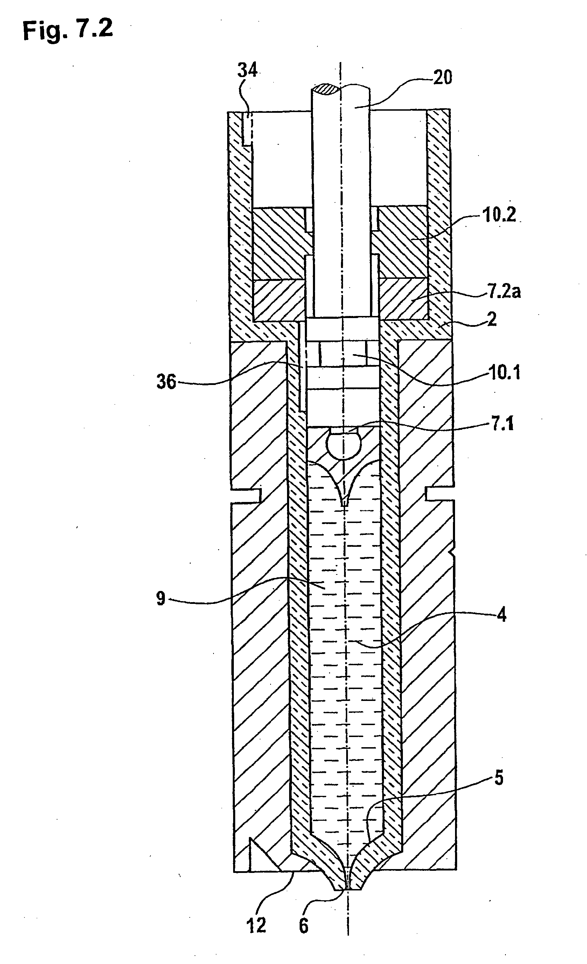

Zum Injizieren der Injektionslösung 4 wird die verrastete Verschlusskappe 11 von dem Außenzylinder 13 abgezogen. Der stumpfe Kegel 29 des Austrittselementes 6 wird nun an bzw. auf den vorher desinfizierten Körperteil gedrückt, in den die Injektionslösung 4 injiziert werden soll. Mittels einer nicht dargestellten Auslösevorrichtung wird die vorgespannte Feder 25 freigegeben, wodurch der Kolbenschieber 20 auf den ersten inneren Kolbenkörper 10.1 des ersten Kolbens 10 schnellt und den ersten inneren Kolbenkörper 10.1 sowie den zweiten inneren Kolbenkörper 7.1 des zweiten Kolbens 7 in der zweiten Kammer 9 in Längsrichtung auf die Stirnwand 5 hin vorschnellen lässt. Die Injektionslösung 4 entweicht mit sehr hoher Geschwindigkeit durch die mindestens eine Düsenbohrung bzw. durch das Austrittelement 6 aus der zweiten Kammer 9, wie in den Figuren 7.1 und 7.2 dargestellt.

Wenn die Injektionslösung 4 vollständig aus der zweiten Kammer 9 gedrückt ist, liegt der zweite innere Kolbenkörper 7.1 an der Stirnwand 5 an und verschließt die Düse(n) 6, wie in den Figuren 8.1 und 8.2 dargestellt.

Weiterhin dargestellt ist in Figur 8.2 eine Ausführung, bei der mindestens ein Gefriertrocknungskanal 16.1 in einem Schlitzring 7.2b ausgebildet ist. Der weiter oben beschriebene Gefriertrocknungskanal 34 wird funktionell durch den dargestellten Gefriertrocknungskanal 16.1 ersetzt. In der Kammer 9 befindet sich mindestens ein Überleitungskanal 36.

Es versteht sich von selbst, dass die Anzahl der Kanäle 16.1, 34 und 36 variieren kann. Anstelle des Überleitungskanals 36 kann in einer weiteren Ausführung auch mindestens ein Überleitungskanal im zweiten äußeren Kolbenring 7.2 ausgebildet sein. Somit besteht die Möglichkeit, die Kanäle, je nach Anforderung bzw. Möglichkeit, entweder nur in der Zylinderwand 35 oder in der Zylinderwand 35 und dem zweiten äußeren Kolbenring 7.2 auszubilden.

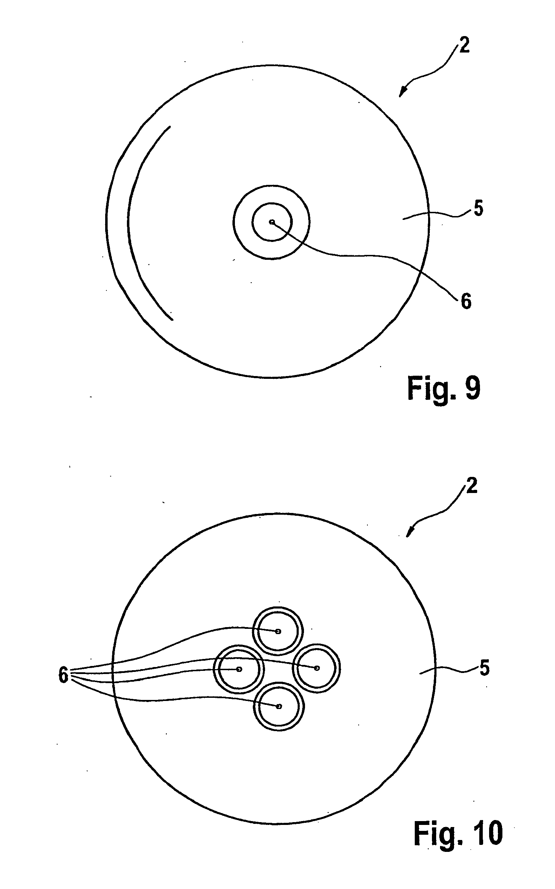

Figur 9 zeigt in Unteransicht, also vom vorderen Ende zum hinteren Ende der Zylinder-Kolben-Einheit 1, eine Version der Zylinder-Kolben-Einheit 1 mit stumpfem Kegel 29 und einer Düse 6.

Figur 10 zeigt in Unteransicht eine Version der Zylinder-Kolben-Einheit 1 mit vier stumpfen Kegeln 29 und vier Düsen 6.

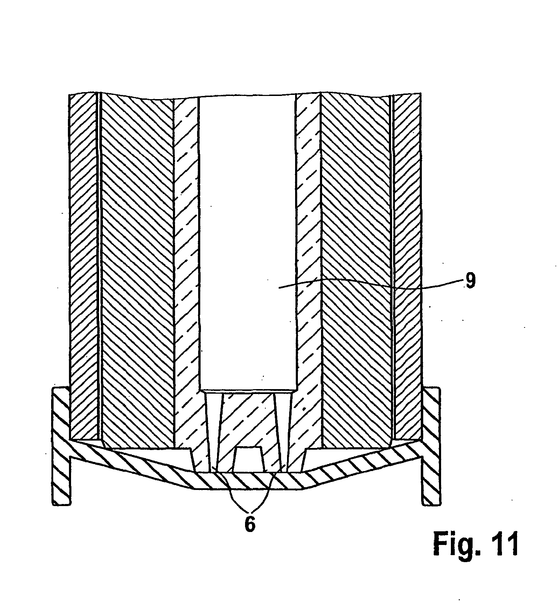

Die Version aus Figur 10 ist in Figur 11 im Querschnitt dargestellt, wobei sich zwei diagonal gegenüberliegende Austrittselemente 6 in der vertikalen Schnittebene befinden.

In den Figuren 12 bis 19 ist der zweite äußere Kolbenring 7.2 mit jeweils unterschiedlich ausgebildetem Überleitungskanal 16 dargestellt. Der zweite innere Kolbenkörper 7.1 ist nicht dargestellt.

Die erste Funktion des in Figur 12 dargestellten zweiten äußeren Kolbenrings 7.2 ist in Fig. 2 zu sehen. Durch den geöffneten Überleitungskanal 16 steht die aus erster Kammer 8 und zweiter Kammer 9 bestehenden Kammer 3 der Zylinder-Kolben-Einheit 1 mit der Umgebung bzw. nach außen in Verbindung, so dass die Gefriertrocknung erfolgen kann. Die zweite Funktion - wasserdampfdichtes Verschließen des Lyophilisats in der zweiten Kammer 9 - ist in den Figuren 3 und 4 zu sehen. Schließlich ist in Figur 5 die dritte Funktion des zweiten äußeren Kolbenringes 7.2 mit dem Überleitungskanal 16, er wird auch als Bypass bezeichnet, zu sehen. Hierdurch gelangt das Lösungsmittel 19, vorzugsweise Wasser, aus der ersten Kammer 8 in die zweite Kammer 9, in der sich das Lyophilisat 17 befindet. Der im Halbschnitt (Oben), in Draufsicht (Mitte) und in Vorderansicht (Unten) - aber um 90 Grad nach vorne gedrehte - dargestellte zweite äußere Kolbenring 7.2 zeigt eine mögliche Ausbildung des Überleitungskanals 16, der hier eine Breite, die kleiner ist als der Innendurchmesser und eine Höhe aufweist, die in etwa 2/3 der Gesamthöhe entspricht.

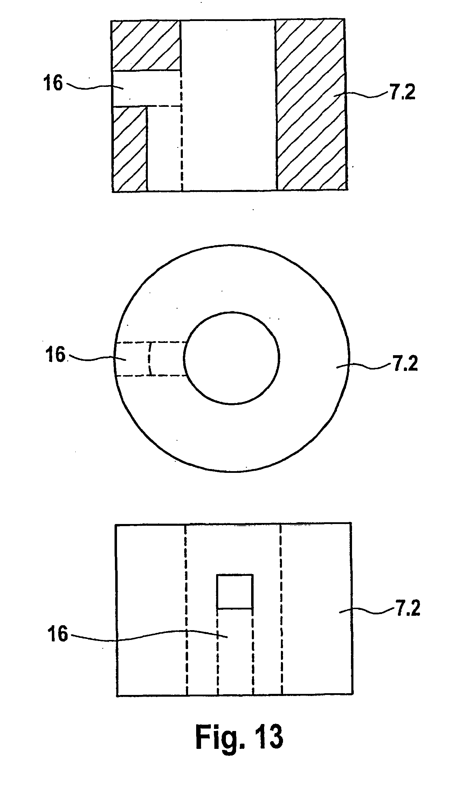

Eine weitere Ausbildung ist in Figur 13 dargestellt. Durch diese Ausgestaltung werden ein geringeres Totvolumen und eine höhere mechanische Stabilität erreicht. Hierzu erhält der zweite äußere Kolbenring 7.2 ein radial nach außen weisendes viereckiges oder alternatives (z.B. rundes) Fenster, etwa in der halben Höhe des zweiten äußeren Kolbenringes 7.2 angeordnet, und einen mit dem Fenster korrespondierenden achsparallelen Kanal, der die Breite des Fensters und eine Tiefe bis zum mittleren Radius aufweist.

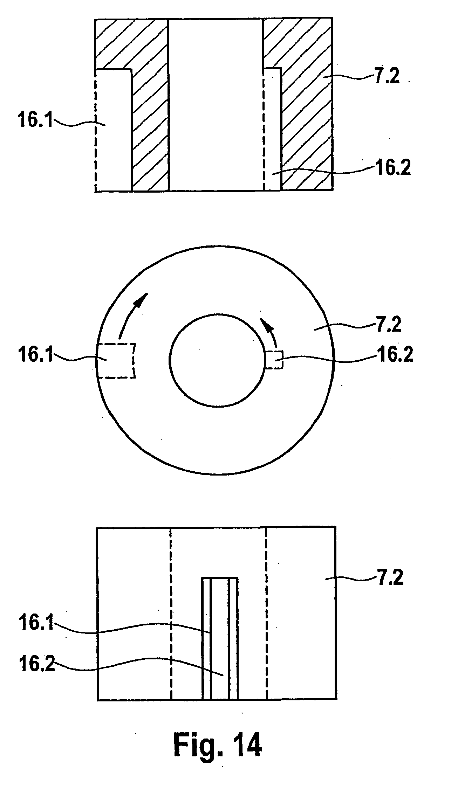

Ein mit zwei funktionell getrennten Überleitungskanälen 16 ausgebildeter zweiter äußerer Kolbenring 7.2 ist in Figur 14 dargestellt. Dabei ist ein äußerer Überleitungskanal 16.1 (Gefriertrocknungskanal) während des Gefriertrocknens und ein innerer Überleitungskanal 16.2 (Überströmkanal) beim Übergang des Lösungsmittels 19 in Funktion. Die beiden Überleitungskanäle 16.1, 16.2 können, in Achsrichtung gesehen, in jedem beliebigen Winkel zueinander stehen, wobei die obere, der Außenseite 12 der Zylinder-Kolben-Einheit 1 entgegen gesetzten Ringfläche geschlossen ist. Die Ausbildung von mehreren der Überleitungskanäle 16.1, 16.2 ist auch möglich. Hierdurch wird beispielsweise die Gefriertrocknungsgeschwindigkeit und die Fließgeschwindigkeit des Lösungsmittels 19 beim Übergang von der ersten Kammer 8 in die zweite Kammer 9 beeinflusst. Die Höhe der Überleitungskanäle 16.1, 16.2 entspricht vorzugsweise 1/2 bis 2/3 der Höhe des zweiten äußeren Kolbenringes 7.2, wobei die Höhen der jeweiligen Überleitungskanäle 16.1, 16.2 nicht gleich hoch sein müssen.

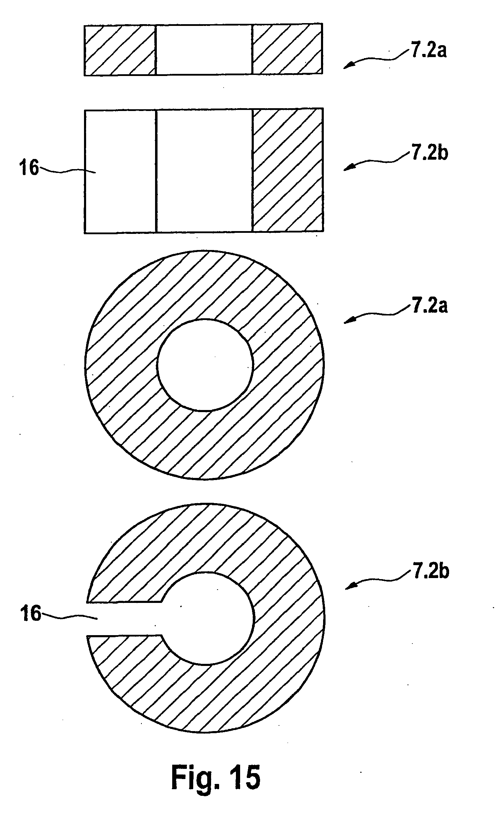

Figur 15 zeigt den zweiten äußeren Kolbenring 7.2 der Figur 12 in zweiteiliger Ausführung. Der obere Teil 7.2a besteht aus einem geschlossenen Quadring (Hohlzylinder); der untere Teil 7.2b aus einem Quadring (Hohlzylinder) mit durchgehendem Schlitz 16. Auch die in den Figuren 13 und 14 gezeigten trifunktionellen Kolbenringe 7.2 können zweiteilig ausgeführt sein.

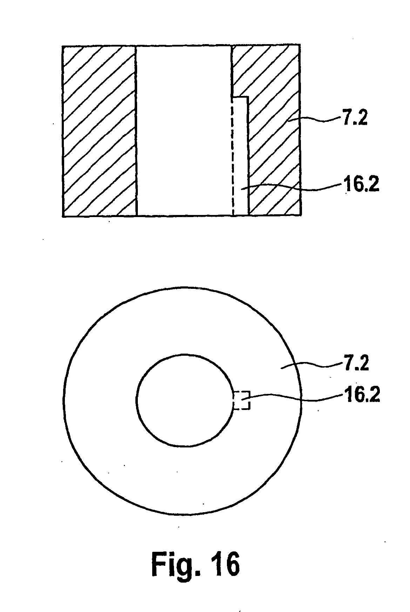

In Figur 16 ist ein zweiter äußerer Kolbenring 7.2 mit einem bifunktionalen Überleitungskanal 16.2 dargestellt, der zusammen mit dem zweiten inneren Kolbenkörper 7.1 mit einer normalen Kolbensetzmaschine nach der Gefriertrocknung gesetzt werden kann. Der zweite innere Kolbenkörper 7.1 mit dem verbundenen zweiten äußeren Kolbenring 7.2 verschließen dann das in der zweiten Kammer 9 vorliegende Lyophilisat 17 analog der Darstellung in Figur 4. Die zweite Funktion als Überleitungskanal 16.2 für das Lösungsmittel 19 ist analog zu Figur 5.

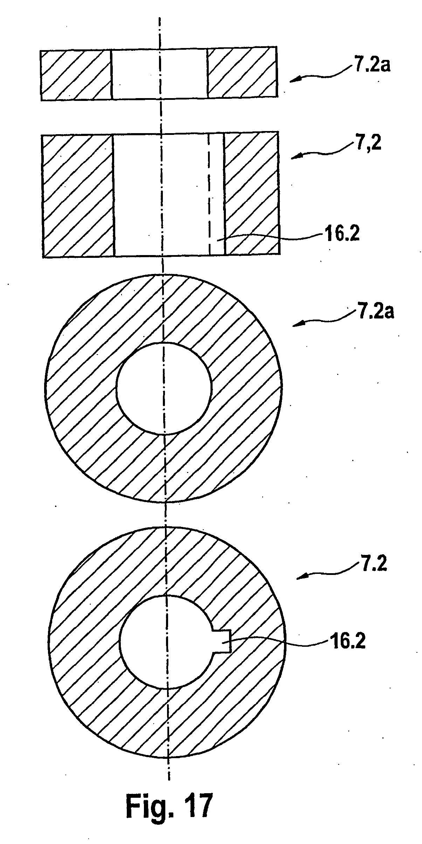

Die Ausbildung des zweiten äußeren Kolbenringes 7.2 der Figur 16 aus zwei Teilen ist in Figur 17 dargestellt.

Selbstverständlich kann man bei allen zweiten äußeren Kolbenringen 7.2 den Überströmkanal 16.2 nicht nur an dem oberen Ende, sondern auch am unteren Ende - also beidseitig - schließen lassen.

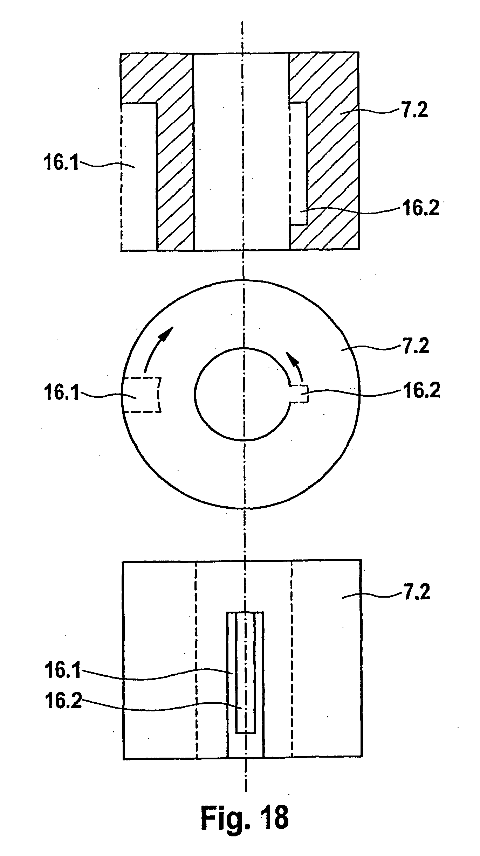

So zeigt Figur 18 den zweiten äußeren Kolbenring 7.2 der Figur 14 bei dem der Überströmkanal 16.2 um ein geschlossenes Stück ergänzt ist. Hingegen muss der Gefriertrocknungskanal 16.1 auch in der Verlängerung bis unten offen weiterlaufen.

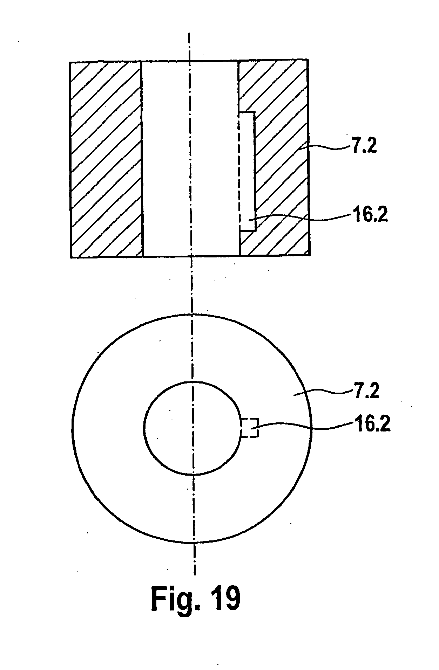

In Figur 19 ist der bifunktionelle Überströmkanal 16.2 der Figur 16 mit einer geschlossenen Verlängerung als Beispiel gezeigt.

### Bezugszeichenliste

- 1.: Zylinder-Kolben-Einheit
- 2.: Zylinder
- 3.: Kammer
- 4.: Injektionslösung
- 5.: Stirnwand
- 6.: Düsenbohrung bzw. Austrittselement
- 7.: zweiter Kolben
- 7.1: zweiter innerer Kolbenkörper
- 7.2: zweiter äußerer Kolbenring
- 7.2a: Quadring
- 7.2b: Schlitzring
- 8.: erste Kammer
- 9.: zweite Kammer
- 10.: erster Kolben
- 10.1: erster innerer Kolbenkörper
- 10.2: erster äußerer Kolbenring
- 11.: Verschlusskappe mit Rastelement
- 12.: Außenseite des druckstabilen Außenzylinders 13
- 13.: Außenzylinder, druckstabil mit Rastnut
- 14.: erster Bestandteil / erste Komponente
- 15.: zweiter Bestandteil / zweite Komponente
- 16.: Überleitungskanal, Überströmkanal, Bypass, Schlitz, kombiniert, trifunktionell
- 16.1: Gefriertrocknungskanal, alternativ im zweiten äußeren Kolbenring 7.2 ergibt Schlitzring 7.2b
- 16.2: Überleitungskanal, Überströmkanal, bifunktionell
- 17.: Lyophilisat
- 18.: Ringfläche
- 19.: Lösungsmittel
- 20.: Kolbenschieber
- 21.: hintere Kante des Kolbenkörpers 7.1
- 22.: Gewindeflansch
- 23.: Gewindebohrung
- 24.: Gewinde
- 25.: Feder
- 26.: Membrane an Verschlusskappe 11
- 27.: Membrane alternativ an Außenzylinder 13
- 28.: Innenseite der Kammer 9 bzw. des Ausströmtrichters 30
- 29.: Kegel, stumpfer
- 30.: Ausströmtrichter
- 31.: Gaspolster, Gasblase
- 32.: Halteelement
- 33.: Federhaken
- 34.: Gefriertrocknungskanal
- 35.: Zylinderwand
- 36.: Überleitungskanal

## Patentansprüche

1. Zylinder-Kolben-Einheit (1) für einen nadelfreien Injektor, beispielsweise zur Bereitstellung einer sterilen Injektionslösung, mit einer in einem Zylinder (2) angeordneten Kammer (3) zur Aufnahme einer Injektionslösung (4), einer Stirnwand (5) mit mindestens einer Düsenbohrung bzw. einem Austrittselement (6) und einem in der Kammer (3) beweglich angeordneten zweiten Kolben (7),
- wobei die Kammer (3) zweiteilig mit einer ersten Kammer (8) und einer konzentrischen zweiten Kammer (9) ausgebildet ist,
- wobei der Querschnitt der ersten Kammer (8) größer ist als der Querschnitt der zweiten Kammer (9),
- wobei die erste Kammer (8) durch einen ersten Kolben (10) und die zweite Kammer (9) durch den zweiten Kolben (7) verschlossen ausgebildet ist,
- wobei die erste Kammer (8) von der zweiten Kammer (9) getrennt ist, **dadurch gekennzeichnet,**
- **dass** der zweite Kolben (7) mit mindestens einem Überleitungskanal (16) oder
- **dass** die erste Kammer (8) mit mindestens einem verschließbaren, axial ausgerichteten Gefriertrocknungskanal (34) und die zweite Kammer (9) mit mindestens einem axial ausgerichteten Überleitungskanal (36), an den jeweils hinteren Enden der Kammern (8, 9) und
**dass** der zweite Kolben (7) als ein innerer zweiter Kolbenkörper (7.1) mit einem äußeren zweiten Kolbenring (7.2) und der erste Kolben (10) als ein innerer erster Kolbenkörper (10.1) mit einem äußeren ersten Kolbenring (10.2) ausgebildet ist.

2. Zylinder-Kolben-Einheit nach Anspruch 1,
**dadurch gekennzeichnet,**
**dass** der innere erste Kolbenkörper (10.1) reibschlüssig mit dem äußeren ersten Kolbenring (10.2) und der innere zweite Kolbenkörper (7.1) reibschlüssig mit dem äußeren zweiten Kolbenring (7.2) verbunden ausgebildet ist.

3. Zylinder-Kolben-Einheit nach einem der vorherigen Ansprüche,
**dadurch gekennzeichnet,**
**dass** zwischen der ersten Kammer (8) und der zweiten Kammer (9) ein Übergangsbereich als Ringfläche (18) ausgebildet ist.

4. Zylinder-Kolben-Einheit nach einem der vorherigen Ansprüche,
**dadurch gekennzeichnet,**
**dass** eine Verschlusskappe (11) mit einer Membrane (26) ausgebildet ist oder alternativ die Membrane (27) separat am Außenzylinder (13) befestigt ist.

5. Zylinder-Kolben-Einheit nach Anspruch 4,
**dadurch gekennzeichnet,**
**dass** die Verschlusskappe (11) direkt oder indirekt mit der Mantelfläche des Außenzylinders (13) verbunden ausgebildet ist.

6. Zylinder-Kolben-Einheit nach einem der vorherigen Ansprüche,
**dadurch gekennzeichnet,**
**dass** der zweite innere Kolbenkörper (7.1) des zweiten Kolbens (7) an der der zweiten Kammer (9) zugewandten Kante mit mindestens einer Dichtungslippe ausgebildet ist.

7. Zylinder-Kolben-Einheit nach einem der vorherigen Ansprüche,
**dadurch gekennzeichnet,**
**dass** der im Lagerzustand verschlossene Überleitungskanal (16, 36) durch Verschieben des zweiten inneren Kolbenkörpers (7.1) geöffnet wird und die erste Kammer (8) mit der zweiten Kammer (9) verbindet.

8. Verfahren zum Befüllen einer Zylinder-Kolben-Einheit (1) für einen nadelfreien Einweginjektor, umfassend mindestens folgende Schritte:
- Befüllen einer zweiten Kammer (9) mit einem ersten Bestandteil / eine ersten Komponente (14) einer Injektionslösung (4), wobei das Volumen des ersten Bestandteils / der ersten Komponente (14) kleiner ist als das Volumen der zweiten Kammer (9);
- Einsetzen eines gequetschten, die Luft entweichen lassenden, zweiten Kolbens (7) in eine erste Kammer (8) bis zur Anlage an eine Ringfläche (18), um die zweite Kammer (9) zu verschließen, wobei in der zweiten Kammer (9) ein Gaspolster (31) vorliegt;
- Befüllen der ersten Kammer (8) oberhalb des zweiten Kolbens (7) mit einem Lösungsmittel (19), z.B. Wasser oder einem zweiten Bestandteil / einer zweiten Komponente (15),
- Einsetzen eines ersten gequetschten Kolbens (10) in die erste Kammer (8) und verschließen der ersten Kammer (8),
- Einschieben des ersten Kolbens (10) mit nach oben zeigender(n) Düse(n) (6) zur Herstellung der Injektionslösung (4) aus dem Lyophilisat bzw. dem ersten Bestandteil / der ersten Komponente (14) und des Lösungsmittels (19) oder des zweiten Bestandteils / der zweiten Komponente (15) durch einen Kolbenschieber (20), wobei durch Überdruck in der ersten Kammer (8) ein zweiter innerer Kolbenkörper (7.1) soweit in einem zweiten äußeren Kolbenring (7.2) in Richtung zweite Kammer (9) geschoben wird, bis ein bisher verschlossener Überleitungskanal (16, 36) die zweite Kammer (9) mit der ersten Kammer (8) verbindet und das Lösungsmittel (19) oder der zweite Bestandteil/ die zweite Komponente (15) vollständig in die zweite Kammer (9) gelangt und sich mit dem Lyophilisat bzw. dem ersten Bestandteil / der ersten Komponente (14) zur fertigen Injektionslösung (4) löst oder mischt;
- nachfolgendes Einschieben eines ersten inneren Kolbenkörpers (10.1) gemeinsam mit dem zweiten inneren Kolbenkörper (7.1) mit Hilfe des Kolbenschiebers (20) durch Eindrehen des Kolbenschiebers (20) in ein Gewinde, Teilgewinde oder Rastelement und Herausdrücken einer Gasblase (31) aus der Zylinder-Kolben-Einheit (1) wobei die Gasblase (31) durch eine oder mehrere nach oben zeigende/-n Düsenbohrung/-en bzw. Austrittselement/-e (6) aus der zweiten Kammer (9) verdrängt wird und eine an einem Außenzylinder (13) oder an einer Verschlusskappe (11) befestigten Membrane (26), die sich bei Austritt des Gaspolsters (31) von den Düsenbohrung/-en bzw. Austrittselement/-en (6) abhebt, und
- nach dem Austritt des Gaspolsters die Düsenbohrung/-en bzw. das/die Austrittselement/-e (6) wieder steril verschließt (Überdruckventil).

9. Verfahren nach Anspruch 8,
**dadurch gekennzeichnet,**
**dass** der erste Bestandteil / die erste Komponente (14) in der zweiten Kammer (9) gefriergetrocknet wird.

10. Verfahren nach Anspruch 8,
**dadurch gekennzeichnet,**
**dass** der vollständige Austritt des Gaspolsters (31) kontrollierbar ist.

## Claims

1. Cylinder-piston unit (1) for a needle-free injector, for example for providing a sterile injection solution, with a chamber (3) which is arranged in a cylinder (2) and is intended for receiving an injection solution (4), an end wall (5) with at least one nozzle bore or an outlet element (6), and a second piston (7) arranged movably in the chamber (3),
- the chamber (3) being of two-part design with a first chamber (8) and a concentric second chamber (9),
- the cross section of the first chamber (8) being larger than the cross section of the second chamber (9),
- the first chamber (8) being designed such that it is closed by a first piston (10) and the second chamber (9) being designed such that it is closed by the second piston (7),
- the first chamber (8) being separated from the second chamber (9), **characterized**
- **in that** the second piston (7) is designed with at least one transfer passage (16), or
- **in that** the first chamber (8) is designed with at least one closable, axially aligned freeze-drying passage (34) and the second chamber (9) is designed with at least one axially aligned transfer passage (36), at the rear ends in each case of the chambers (8, 9), and
- **in that** the second piston (7) is designed as an inner second piston body (7.1) with an outer second piston ring (7.2), and the first piston (10) is designed as an inner first piston body (10.1) with an outer first piston ring (10.2).

2. Cylinder-piston unit according to Claim 1, **characterized in that** the inner first piston body (10.1) is designed such that it is connected frictionally to the outer first piston ring (10.2), and the inner second piston body (7.1) is designed such that it is connected frictionally to the outer second piston ring (7.2).

3. Cylinder-piston unit according to one of the preceding claims, **characterized in that** a transition region is designed as an annular surface (18) between the first chamber (8) and the second chamber (9).

4. Cylinder-piston unit according to one of the preceding claims, **characterized in that** a closure cap (11) is designed with a membrane (26) or alternatively the membrane (27) is fastened separately to the outer cylinder (13).

5. Cylinder-piston unit according to Claim 4, **characterized in that** the closure cap (11) is designed such that it is connected directly or indirectly to the circumferential surface of the outer cylinder (13).

6. Cylinder-piston unit according to one of the preceding claims, **characterized in that** the second inner piston body (7.1) of the second piston (7) is designed with at least one sealing lip on the edge facing the second chamber (9).

7. Cylinder-piston unit according to one of the preceding claims, **characterized in that** the transfer passage (16, 36) which is closed in the storage state is opened by displacement of the second inner piston body (7.1) and connects the first chamber (8) to the second chamber (9).

8. Method for filling a cylinder-piston unit (1) for a needle-free disposable injector, comprising at least the following steps:
- filling a second chamber (9) with a first component (14) of an injection solution (4), wherein the volume of the first component (14) is smaller than the volume of the second chamber (9);
- inserting a second piston (7), which is compressed and allows the air to escape, into a first chamber (8) until it makes contact with an annular surface (18) in order to close the second chamber (9), the second chamber (9) containing a gas cushion (31);
- filling the first chamber (8) above the second piston (7) with a solvent (19), for example water, or with a second component (15),
- inserting a first compressed piston (10) into the first chamber (8) and closing the first chamber (8),
- pushing in the first piston (10), with nozzle(s) (6) pointing upward in order to produce the injection solution (4) from the lyophilizate or the first component (14), and the solvent (19) or the second component (15) by means of a piston slide (20), wherein, by means of positive pressure in the first chamber (8), a second inner piston body (7.1) is pushed in a second outer piston ring (7.2) in the direction of a second chamber (9) until a previously closed transfer passage (16, 36) connects the second chamber (9) to the first chamber (8) and the solvent (19) or the second component (15) entirely enters the second chamber (9) and is dissolved or mixed with the lyophilizate or the first component (14) to provide the finished injection solution (4);
- subsequently pushing in a first inner piston body (10.1) together with the second inner piston body (7.1) with the aid of the piston slide (20), by screwing the piston slide (20) into a thread, partial thread or latching element, and squeezing a gas bubble (31) out of the cylinder-piston unit (1), wherein the gas bubble (31) is displaced out of the second chamber (9) through one or more upwardly pointing nozzle bore(s) or outlet element(s) (6) and a membrane (26) which is fastened to an outer cylinder (13) or to a closure cap (11) and lifts off from the nozzle bore(s) or outlet element(s) (6) when the gas cushion (31) emerges, and
- closes the nozzle bore(s) or the outlet element(s) (6) again in a sterile manner (pressure control valve) after the gas cushion emerges.

9. Method according to Claim 8, **characterized in that** the first component (14) is freeze-dried in the second chamber (9).

10. Method according to Claim 8, **characterized in that** the full emergence of the gas cushion (31) is checkable.

## Revendications

1. Unité cylindre/piston (1) pour un injecteur sans aiguille, par exemple pour fournir une solution injectable stérile, comprenant une chambre (3) disposée dans un cylindre (2) pour recevoir une solution injectable (4), une paroi frontale (5) comprenant au moins un alésage de buse ou un élément de sortie (6) et un deuxième piston (7) disposé de manière mobile dans la chambre (3),
- la chambre (3) étant réalisée en deux parties avec une première chambre (8) et une deuxième chambre concentrique (9),
- la section transversale de la première chambre (8) étant plus grande que la section transversale de la deuxième chambre (9),
- la première chambre (8) étant réalisée de manière à être fermée par un premier piston (10) et la deuxième chambre (9) étant réalisée de manière à être fermée par le deuxième piston (7),
- la première chambre (8) étant séparée de la deuxième chambre (9),
**caractérisée**
- **en ce que** le deuxième piston (7) est réalisé avec au moins un canal de transfert (16) ou
- **en ce que** la première chambre (8) est réalisée avec au moins un canal de lyophilisation (34) orienté axialement et pouvant être fermé et la deuxième chambre (9) est réalisée avec au moins un canal de transfert (36) orienté axialement, aux extrémités arrière respectives des chambres (8, 9) et
- **en ce que** le deuxième piston (7) est réalisé sous la forme d'un deuxième corps de piston intérieur (7.1) avec une deuxième bague de piston extérieure (7.2) et le premier piston (10) est réalisé sous la forme d'un premier corps de piston intérieur (10.1) avec une première bague de piston extérieure (10.2).

2. Unité cylindre/piston selon la revendication 1,
**caractérisée**
**en ce que** le premier corps de piston intérieur (10.1) est réalisé de manière à être relié par engagement par friction à la première bague de piston extérieure (10.2) et le deuxième corps de piston intérieur (7.1) est réalisé de manière à être relié par engagement par friction à la deuxième bague de piston extérieure (7.2).

3. Unité cylindre/piston selon l'une quelconque des revendications précédentes,
**caractérisée**
**en ce qu'**une région de transition est réalisée sous forme de surface annulaire (18) entre la première chambre (8) et la deuxième chambre (9).

4. Unité cylindre/piston selon l'une quelconque des revendications précédentes,
**caractérisée**
**en ce qu'**un capuchon de fermeture (11) est réalisé avec une membrane (26) ou en variante la membrane (27) est fixée séparément au cylindre extérieur (13).

5. Unité cylindre/piston selon la revendication 4,
**caractérisée**
**en ce que** le capuchon de fermeture (11) est réalisé de manière à être relié directement ou indirectement à la surface d'enveloppe du cylindre extérieur (13).

6. Unité cylindre/piston selon l'une quelconque des revendications précédentes,
**caractérisée**
**en ce que** le deuxième corps de piston intérieur (7.1) du deuxième piston (7) est réalisé avec au moins une lèvre d'étanchéité sur l'arête tournée vers la deuxième chambre (9).

7. Unité cylindre/piston selon l'une quelconque des revendications précédentes,
**caractérisée**
**en ce que** le canal de transfert (16, 36) fermé dans l'état de stockage est ouvert par coulissement du deuxième corps de piston intérieur (7.1) et relie la première chambre (8) à la deuxième chambre (9).

8. Procédé de remplissage d'une unité cylindre/piston (1) pour un injecteur sans aiguille à usage unique, comportant au moins les étapes suivantes :
- remplissage d'une deuxième chambre (9) d'un premier constituant/d'un premier composant (14) d'une solution injectable (4), le volume du premier constituant/du premier composant (14) étant inférieur au volume de la deuxième chambre (9) ;
- insertion d'un deuxième piston (7) comprimé et laissant s'échapper l'air dans une première chambre (8) jusqu'à ce qu'il vienne s'appliquer contre une surface annulaire (18), afin de fermer la deuxième chambre (9), un coussin de gaz (31) étant présent dans la deuxième chambre (9) ;
- remplissage de la première chambre (8) au-dessus du deuxième piston (7) d'un solvant (19), par exemple d'eau, ou d'un deuxième constituant/d'un deuxième composant (15),
- insertion d'un premier piston comprimé (10) dans la première chambre (8) et fermeture de la première chambre (8),
- introduction du premier piston (10) avec une/des buse (s) (6) tournée (s) vers le haut pour produire la solution injectable (4) à partir du lyophilisat ou du premier constituant/du premier composant (14) et du solvant (19) ou du deuxième constituant/du deuxième composant (15) au moyen d'un poussoir de piston (20), un deuxième corps de piston intérieur (7.1) étant poussé, par surpression dans la première chambre (8), dans une deuxième bague de piston extérieure (7.2) dans la direction de la deuxième chambre (9) jusqu'à ce qu'un canal de transfert (16, 36) jusqu'alors fermé relie la deuxième chambre (9) à la première chambre (8) et que le solvant (19) ou le deuxième constituant/le deuxième composant (15) parvienne complètement dans la deuxième chambre (9) et se dissolve ou se mélange avec le lyophilisat ou le premier constituant/le premier composant (14) pour produire la solution injectable finie (4) ;
- introduction subséquente d'un premier corps de piston intérieur (10.1) conjointement avec le deuxième corps de piston intérieur (7.1) à l'aide du poussoir de piston (20) par vissage du poussoir de piston (20) dans un filetage, un filetage partiel ou un élément d'encliquetage et poussée d'une bulle de gaz (31) hors de l'unité cylindre/piston (1), la bulle de gaz (31) étant chassée hors de la deuxième chambre (9) à travers un ou plusieurs alésages de buse ou éléments de sortie (6) tournés vers le haut et une membrane (26) fixée à un cylindre extérieur (13) ou à un capuchon de fermeture (11), la membrane se soulevant du ou des alésages de buse ou du ou des éléments de sortie (6) lors de la sortie du coussin de gaz (31), et
- après la sortie du coussin de gaz, fermant à nouveau de manière stérile (soupape de surpression) le(s) alésage(s) de buse ou le(s) élément(s) de sortie (6).

9. Procédé selon la revendication 8,
**caractérisé**
**en ce que** le premier constituant/le premier composant (14) est lyophilisé dans la deuxième chambre (9).

10. Procédé selon la revendication 8,
**caractérisé**
**en ce que** la sortie complète du coussin de gaz (31) peut être contrôlée.
